# EUROPÄISCHE PATENTSCHRIFT

(11) **EP 3 801 665 B1**
(45) Veröffentlichungstag und Bekanntmachung des Hinweises auf die Patenterteilung: **06.07.2022**
(21) Anmeldenummer: 19729210.5
(22) Anmeldetag: 30.05.2019
(51) Int. Cl.: A61M 60/135, A61M 60/148, A61M 60/205

(54) **AXIALPUMPE FÜR EIN HERZUNTERSTÜTZUNGSSYSTEM UND VERFAHREN ZUM HERSTELLEN EINER AXIALPUMPE FÜR EIN HERZUNTERSTÜTZUNGSSYSTEM**
AXIAL-FLOW PUMP FOR A VENTRICULAR ASSIST DEVICE AND METHOD FOR PRODUCING AN AXIAL-FLOW PUMP FOR A VENTRICULAR ASSIST DEVICE
POMPE AXIALE POUR UN DISPOSITIF D'ASSISTANCE VENTRICULAIRE ET PROCÉDÉ POUR FABRIQUER UNE POMPE AXIALE POUR UN DISPOSITIF D'ASSISTANCE VENTRICULAIRE

(30) Priorität: 30.05.2018 DE 102018208541
(43) Veröffentlichungstag der Anmeldung: 14.04.2021
(73) Patentinhaber: Kardion GmbH, 70376 Stuttgart (DE)
(72) Erfinder: STOTZ, Ingo, 71254 Ditzingen (DE); BAEUERLE, Niko, 75378 Bad Liebenzell (DE)
(74) Vertreter: Gauss, Nikolai
(86) Internationale Anmeldenummer: PCT/EP2019/064157
(87) Internationale Veröffentlichungsnummer: WO 2019/229223

(56) Entgegenhaltungen:
- US-A1- 2009 138 080
- US-A1- 2015 051 438
- US-A1- 2016 144 089

## Beschreibung

Die Offenbarung betrifft eine Axialpumpe für ein Herzunterstützungssystem und ein Verfahren zum Herstellen einer Axialpumpe für ein Herzunterstützungssystem. Darüber hinaus betrifft die Offenbarung eine Vorrichtung für das Herstellen einer Axialpumpe für ein Herzunterstützungssystem sowie ein Computerprogramm für das Ausführen und/oder Ansteuern eines solchen Herstellungsverfahrens.

Zur Herz-Kreislauf-Unterstützung herzinsuffizienter Patienten werden unter anderem Systeme eingesetzt, die einen Teil oder die komplette Pumpfunktion des Herzens übernehmen. Diese Systeme, auch Herzunterstützungssysteme oder kurz VAD (ventricular assist device) genannt, können in temporäre Systeme zur kurzeitigen Herzunterstützung und dauerhafte Systeme zum langzeitigen Verbleib am oder im Patienten untergliedert werden. Bestandteil eines solchen Systems ist in der Regel eine Blutpumpe, typischerweise eine Kreiselpumpe (Turbopumpe), die durch einen integrierten Elektromotor angetrieben wird und mittels eines Laufrades den geforderten Blutfluss erzeugt. Die Pumpe kann hierbei an unterschiedlichen Stellen implantiert werden. Beispielsweise kann die Pumpe durch eine invasive Operation per Sternotomie von außen an das Herz angenäht werden oder minimalinvasiv durch einen Katheter in der Aorta oder im Ventrikel abgesetzt werden. Im letzteren Fall ist der maximal zulässige Außendurchmesser der Pumpe in der Regel auf 10 mm limitiert, weshalb der Einsatz einer Pumpe axialer Bauart mit einem axial angeströmten Laufrad anzustreben ist. Dabei wird das zu fördernde Blut durch am Umfang eines zylindrischen Pumpengehäuses angebrachte Austrittsöffnungen ausgestoßen, um wieder der Aorta zugeführt zu werden.

Bei einer Kreiselpumpe wird durch die Rotation eines Laufrades Drall in die Strömung eingebracht. Der Drall kann beispielsweise durch ein nachgeschaltetes stehendes Leitrad, auch Stator genannt, reduziert werden.

Aufgabe der Erfindung ist es, ein Herzunterstützungssystem bereitzustellen, das eine Axialpumpe umfasst, die Blut sowohl effizient als auch schonend fördern kann.

Diese Aufgabe wird durch ein Herzunterstützungssystem gemäß Anspruch 1 gelöst. Vorteilhafte Ausführungsformen der Erfindung sind in den abhängigen Ansprüchen angegeben.

Nachfolgend werden eine Axialpumpe eines solchen Herzunterstützungssystems, ein Verfahren zum Herstellen der Axialpumpe eines solchen Herzunterstützungssystems, eine Vorrichtung, die dieses Verfahren verwendet, und ein Computerprogramm für das Ausführen und/oder Ansteuern eines solchen Herstellungsverfahrens erläutert.

Der Erfindung beruht auf der Erkenntnis, dass durch eine zumindest abschnittsweise wellenförmig gekrümmte Schaufel an einem Laufrad für eine axiale Kreiselpumpe eines Herzunterstützungssystems in Verbindung mit einer geeigneten Positionierung des Laufrads relativ zu Austrittsöffnungen an einem Pumpengehäuse eine besonders schonende und effiziente Fluidförderung erreicht werden kann.

Die Art der Formgebung des Laufrades führt zu einem gesteigerten Wirkungsgrad bei reduzierter Blutschädigung. Unter gewissen Randbedingungen, etwa bei beschränktem Bauraum, ist es sinnvoll, auf ein feststehendes Leitrad zu verzichten, was in der Regel eine entsprechende Reduktion des Wirkungsgrades der Pumpe mit sich bringt. Durch die Formgebung des Laufrades bzw. dessen Schaufeln und dessen geeignete Positionierung relativ zum Pumpengehäuse ist es nun möglich, die Reduktion des Wirkungsgrades zumindest teilweise zu kompensieren. Weiterhin können dadurch Blutschädigungen durch Hämolyse reduziert werden.

Das erfindungsgemäße Herzunterstützungssystem umfasst die in Anspruch 1 definierten technischen Merkmale. Die Axialpumpe weist folgende Merkmale auf:
ein Pumpengehäuse zur Anordnung in einem Blutgefäß; und
einen in dem Pumpengehäuse um eine Drehachse drehbar gelagerten oder lagerbaren Pumpenläufer bestehend aus einer Nabe und zumindest einem zumindest abschnittsweise schraubenförmig um die Nabe gewundenen Schaufelelement zum Fördern eines Fördermediums in Richtung der Drehachse, wobei das Schaufelelement zumindest einen Schaufelabschnitt mit einer wellenförmigen Schaufelkrümmung aufweist.

Unter einer Axialpumpe kann eine Kreiselpumpe mit integriertem Elektromotor zum axialen Ansaugen eines Fördermediums verstanden werden. Ein Ausstoßen des angesaugten Fördermediums kann beispielsweise in radialer oder diagonaler Richtung durch eine oder mehrere seitliche Austrittsöffnungen im Pumpengehäuse erfolgen. Unter einem Herzunterstützungssystem kann insbesondere eine minimalinvasiv durch einen Katheter in der Aorta oder in einer Herzkammer platzierbare, rohr- oder schlauchförmige Pumpvorrichtung verstanden werden. Beispielsweise kann das Herzunterstützungssystem einen maximalen Außendurchmesser zwischen 10 und 15 mm aufweisen. Unter einem Pumpengehäuse kann ein Gehäuse in Form eines Schlauches oder Rohres verstanden werden. Unter einem Blutgefäß kann eine (Haupt-)Schlagader oder eine Herzkammer verstanden werden. Unter einem Fördermedium kann dementsprechend Blut verstanden werden. Unter einem Pumpenläufer kann ein axial anströmbares Laufrad der Axialpumpe verstanden werden. Der Pumpenläufer kann beispielsweise zwei oder mehr Schaufelelemente aufweisen, die zumindest abschnittsweise schraubenförmig um die Nabe gewunden sein können und zumindest einen Schraubenabschnitt mit einer wellenförmigen Schaufelkrümmung aufweisen können. Unter einer wellenförmigen Schaufelkrümmung kann eine Wellenform einer Skelettlinie des Schaufelelements verstanden werden, die durch zumindest einen Wellenbauch und zumindest ein Wellental charakterisiert ist.

Das Schaufelelement wird durch eine oder mehrere Skelettlinie mit je zumindest einem Wendepunkt und/oder eine lokal konstante oder variable Aufdickung beschrieben oder definiert, insbesondere wobei eine Tangentensteigung einer eine Krümmung der Skelettlinie repräsentierenden Tangente in Strömungsrichtung zunächst zunimmt und ab dem Wendepunkt wieder abnimmt, um die wellenförmige Schaufelkrümmung zu erzeugen. Dadurch kann die wellenförmige Schaufelkrümmung in Richtung der Skelettlinie erzeugt werden.

Unter der Skelettlinie eines Schaufelelements ist vorliegend insbesondere die Mittellinie eines Profils des Schaufelelements in einer zu der Drehachse des Pumpenläufers koaxialen Mantelfläche eines Zylinders zu verstehen, welche die Mittelpunkte aller Kreise verbindet, die in das Profil passen, also die Kurve, die überall zur Ober- und Unterseite des Profils den gleichen (Quer-)Abstand hat.

Die Mantelfläche des Pumpengehäuses weist zumindest eine Austrittsöffnung zum seitlichen Ausleiten des Fördermediums auf. Dabei kann der Schaufelabschnitt im eingebauten Zustand des Pumpenläufers der Austrittsöffnung zumindest teilweise gegenüberliegen. Dadurch kann ein effizientes und schonendes radiales oder diagonales Ausleiten des Fördermediums gewährleistet werden.

Der Wendepunkt liegt etwa im Bereich der Austrittsöffnung, insbesondere im Bereich einer im Betrieb der Axialpumpe stromaufwärts liegenden Kante der Austrittsöffnung, also beispielsweise dem Beginn der Austrittsöffnung. Dadurch kann die Effizienz der Axialpumpe weiter gesteigert werden.

Das Schaufelelement ist ausgehend von einem Anfang des Pumpenläufers schraubenförmig um die Nabe gewunden, wobei die Tangentensteigung ausgehend von dem Anfang des Pumpenläufers zunimmt und ab dem Wendepunkt wieder abnimmt. Eine solche Ausführungsform steigert besonders gut den Wirkungsgrad der Axialpumpe bei reduzierter Blutschädigung.

Gemäß einer weiteren Ausführungsform kann die wellenförmige Schaufelkrümmung in Richtung einer radialen Ausdehnung des Schaufelelements variieren. Unter einer radialen Ausdehnung kann eine Ausdehnung in radialer Richtung ausgehend von der Drehachse verstanden werden. Dadurch kann die Schaufelkrümmung in mehreren Richtungen optimal an verschiedene Randbedingungen angepasst werden.

Gemäß einer weiteren Ausführungsform kann die wellenförmige Schaufelkrümmung mit zunehmendem Abstand von der Drehachse zunehmen. Somit kann eine größtmögliche Effizienz der Axialpumpe erreicht werden.

Es ist ferner von Vorteil, wenn die Nabe einen in Strömungsrichtung zunehmenden Durchmesser aufweist. Dadurch kann das schonende Ausleiten des Fördermediums in radialer oder diagonaler Richtung zur Drehachse unterstützt werden.

Der hier vorgestellte Ansatz schafft zudem ein Verfahren zum Herstellen einer Axialpumpe für ein Herzunterstützungssystem, wobei das Verfahren folgende Schritte umfasst:
Bilden eines Pumpengehäuses zur Anordnung in einem Blutgefäß und
eines Pumpenläufers bestehend aus einer Nabe und zumindest einem zumindest abschnittsweise schraubenförmig um die Nabe gewundenen Schaufelelement zum Fördern eines Fördermediums, wobei das Schaufelelement zumindest einen Schaufelabschnitt mit einer wellenförmigen Schaufelkrümmung aufweist; und
Anordnen des Pumpenläufers in dem Pumpengehäuse, wobei der Pumpenläufer um eine Drehachse drehbar gelagert wird, um das Fördermedium in Richtung der Drehachse zu fördern.

Dieses Verfahren kann beispielsweise in Software oder Hardware oder in einer Mischform aus Software und Hardware, beispielsweise in einem Steuergerät, implementiert sein.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Vorteilhafte Ausführungsbeispiele der Erfindung sind in den Zeichnungen schematisch dargestellt und in der nachfolgenden Beschreibung näher erläutert.

Es zeigen:
- Fig. 1: ein Herzunterstützungssystem mit einer Axialpumpe gemäß einem Ausführungsbeispiel;
- Fig. 2: die Axialpumpe des Herzunterstützungssystems aus Fig. 1 mit Schaufeelementen und mit einer Nabe in einer Seitenansicht;
- Fig. 3: eine Abwicklung eines Schaufelelements der Axialpumpe aus den Figuren 1 und 2;
- Fig. 4: eine perspektivische Teilansicht der Axialpumpe aus den Figuren 1 und 2 mit einem Schaufelelement, das einen Schaufelabschnitt mit einer wellenförmigen Schaufelkrümmung hat und zum Vergleich die Geometrie eines herkömmlichen Schaufelelements für eine Axialpumpe, das keinen wellenförmig gekrümmten Abschnitt aufweist;
- Fig. 5: eine Ansicht des Schaufelelements der in Fig. 1 und Fig. 2 gezeigten Axialpumpe mit auf die die Oberfläche der Nabe bezogenen unterschiedlichen Schaufelhöhen;
- Fig. 6: den Verlaufs des zwischen der Umfangsrichtung und einer Skelettlinie des Schaufelelements gebildeten Schaufelwinkels β entlang einer normierten meridionalen Koordinate m/mMax;
- Fig. 7: ein Ablaufdiagramm eines Verfahrens zum Herstellen einer Axialpumpe gemäß einem Ausführungsbeispiel; und
- Fig. 8: eine schematische Darstellung einer Vorrichtung zum Ausführen und/oder Ansteuern des Verfahrens aus Fig. 7.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

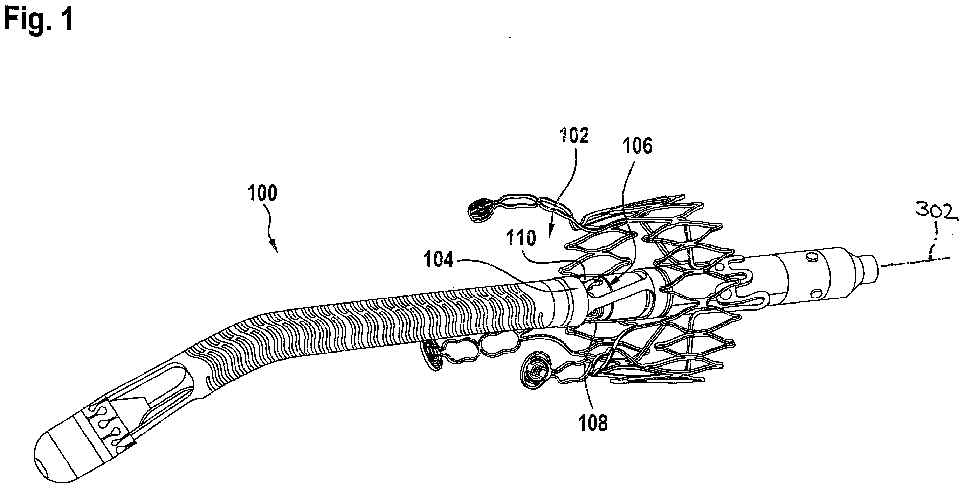

Fig. 1 zeigt eine schematische Darstellung eines Herzunterstützungssystems 100 mit einer Axialpumpe 102 gemäß einem Ausführungsbeispiel. Das Herzunterstützungssystem 100 weist eine rohr- oder schlauchförmige Gestalt auf. Beispielhaft ist das Herzunterstützungssystem 100 als ein minimalinvasiv durch einen Katheter in ein Blutgefäß wie etwa die Aorta oder eine Herzkammer einführbares axiales Kreiselpumpensystem realisiert. Die Axialpumpe 102 hat eine Drehachse 302 und umfasst ein Pumpengehäuse 104, das als ein rohrförmiger Abschnitt des Herzunterstützungssystems 100 realisiert ist, der die Drehachse 302 umgibt und beispielhaft drei einander gegenüberliegende seitliche Austrittsöffnungen 106 zum seitlichen Ausleiten eines Fördermediums, hiervon Blut, aufweist. Gemäß alternativen Ausführungsbeispielen weist das Pumpengehäuse 104 nur eine, zwei, drei oder auch mehr als drei in Umfangsrichtung verteilte Austrittsöffnungen 106 auf. In dem Pumpengehäuse 104 drehbar gelagert ist ein Pumpenläufer 108, auch Laufrad genannt, der in dem in Fig. 1 gezeigten zusammengebauten Zustand der Axialpumpe 102 abschnittsweise den Austrittsöffnungen 106 gegenüberliegt. Der Pumpenläufer 108 dient zum axialen Ansaugen und radialen oder diagonalen Ausstoßen des Blutes über die Austrittsöffnungen 106.

Um einen möglichst effizienten und schonenden Transport des Blutes zu gewährleisten, weist der Pumpenläufer 108 zumindest ein schraubenförmig gewundenes Schaufelelement 110 auf. Die Skelettlinie des Schaufelelements 110 weist hierbei im Bereich des stromauf liegenden Beginns der Austrittsöffnungen 106 einen Wendepunkt auf.

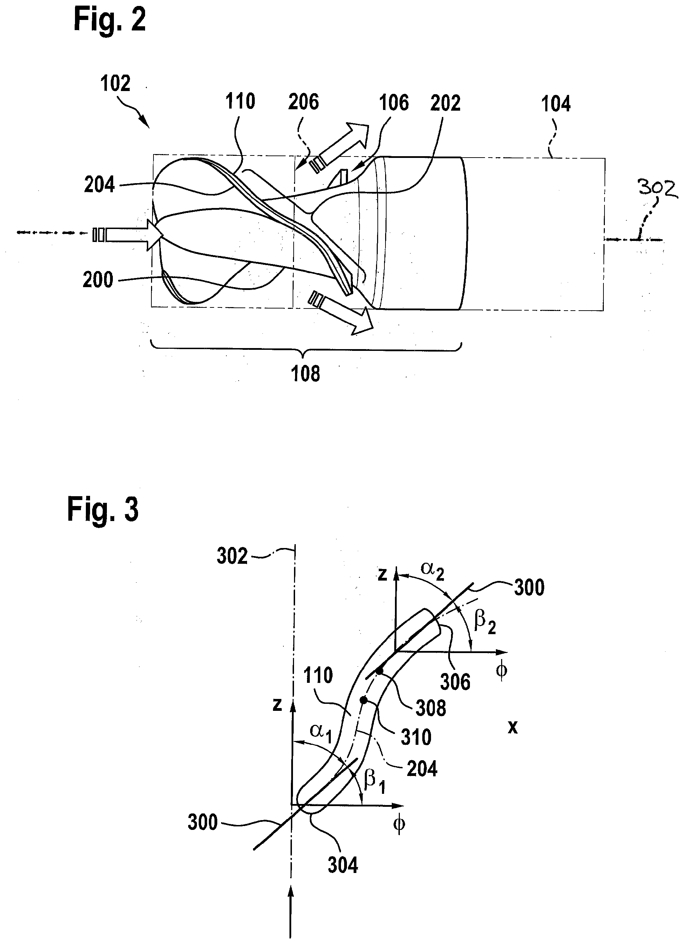

Fig. 2 zeigt eine schematische Darstellung der Axialpumpe 102 aus Fig. 1 in der Seitenansicht. Zu erkennen ist das Schaufelelement 110, das um eine Nabe 200 der Axialpumpe 102 schraubenförmig gewunden ist. Die Nabe 200 bildet dabei einen inneren Kern des Pumpenläufers 108. Eine Strömungsrichtung des Fördermediums ist mit drei Pfeilen schematisch angedeutet. Das Ansaugen des Fördermediums erfolgt durch eine dem Pumpenläufer 108 vorgeschaltete stirnseitige als eine Ansaugöffnung wirkende Eintrittsöffnung im Pumpengehäuse 104.

Gemäß diesem Ausführungsbeispiel erstreckt sich das Schaufelelement 110 ausgehend von einem stromauf liegenden Ende des Pumpenläufers 108 über eine gesamte Länge oder zumindest über einen größten Teil der Nabe 200. Dabei weist die Nabe 200 einen in Strömungsrichtung zunehmenden Durchmesser auf, sodass sich eine in Strömungsrichtung dicker werdende Gestalt der Nabe 200 ergibt. Dadurch wird das radiale bzw. diagonale Ausleiten des Fördermediums erleichtert.

Das Schaufelelement 110 weist einen Schaufelabschnitt 202 mit einer wellenförmigen Schaufelkrümmung auf, die durch eine mehrfache Krümmung einer Skelettlinie 204 des Schaufelelements 110 definiert ist. Unter einer wellenförmigen Schaufelkrümmung ist hierbei eine mit zumindest einem Vorzeichenwechsel verbundene Krümmungsänderung des Schaufelabschnitts 202 zu verstehen.

Wie aus Fig. 2 ersichtlich, befindet sich im eingebauten Zustand des Pumpenläufers 108 zumindest ein Teilabschnitt des Schaufelabschnitts 202 gegenüber den Austrittsöffnungen 106.

Gemäß diesem Ausführungsbeispiel befindet sich der Schaufelabschnitt 202 zumindest teilweise im Bereich einer strömungszugewandten Kante 206 der Austrittsöffnung 106. Der Schaufelabschnitt 202 repräsentiert dabei einen Übergang zwischen einer konvexen und einer konkaven Wölbung.

Beispielhaft umfasst der Pumpenläufer 108 gemäß Fig. 2 zwei Schaufelelemente 110, die gleichsinnig um die Nabe 200 gewunden sind und je den Schaufelabschnitt 202 aufweisen. Gemäß einem alternativen Ausführungsbeispiel ist der Pumpenläufer 108 mit mehr als zwei solcher Schaufelelemente 110 realisiert.

Fig. 3 zeigt eine schematische Darstellung der Abwicklung einer Skelettlinie 204 eines Schaufelelements 110 aus den Figuren 1 und 2. Eingezeichnet sind beispielhaft zwei Schaufelwinkelpaare α₁, β₁ und α₂, β₂, die je eine Tangentensteigung einer die Krümmung der Skelettlinie 204 repräsentierenden Tangente 300 repräsentieren. Die Tangente 300 ist je in einem zylindrischen Koordinatensystem mit einer zu einer Drehachse 302 des Pumpenläufers parallelen z-Achse und einer zur z-Achse senkrechten Φ-Achse eingezeichnet. Die Φ -Achse repräsentiert eine Umfangsrichtung des Pumpenläufers.

Wie aus Fig. 3 ersichtlich, wird die Tangentensteigung in Strömungsrichtung, angedeutet durch einen vertikalen Pfeil, zunächst größer und nimmt anschließend wieder ab.. Gemäß diesem Ausführungsbeispiel nimmt die Tangentensteigung ausgehend von einer Schaufelvorderkante 304 bis zu einer Schaufelhinterkante 306 des Schaufelelements 110 zunächst kontinuierlich zu und bei Erreichen des Wendepunktes 310 der Skelelettlinie 204 wieder ab. Ein Punkt 308 markiert eine Position eines Strömungsaustritts über die Austrittsöffnungen des Pumpengehäuses, genauer einen Beginn des Strömungsaustritts in axialer Richtung. Hierbei ist es anzustreben, dass der Wendepunkt 310 und der Punkt 308 des Beginns des Strömungsaustritts in räumlicher Nähe sind.

Wie bereits beschrieben, ist der Pumpenläufer gemäß einem Ausführungsbeispiel mit mindestens zwei Schaufelelementen 110 realisiert. Das Fördermedium wird dem Pumpenläufer axial zugeführt oder davon angesaugt und radial und diagonal durch eine oder mehrere Austrittsöffnungen im Pumpengehäuse ausgestoßen. Die Schaufelelemente 110 sind dabei so gestaltet, dass sich der Winkel α zwischen der mit einer Schaufeloberfläche oder der Skelettlinie 204 gebildeten Tangente 300 und der Drehachse 302 bzw. der z-Achse in axialer Richtung ändert. In entgegengesetztem Maße ändert sich der Winkel β zwischen der Umfangsrichtung bzw. der Φ-Achse und der Schaufeloberfläche oder der Skelettlinie 204. Der Winkel β ändert sich derart, dass er zumindest im Bereich des größten Durchmessers des Pumpenläufers, also in einem Schnitt im Bereich der Schaufelspitzen der Schaufelelemente 110, vom Beginn des Pumpenläufers an, also ab der Schaufelvorderkante 304, in Strömungsrichtung größer wird. Insbesondere nimmt der Winkel β im Bereich des Beginns des Strömungsaustritts 308 bzw. in räumlicher Nähe zu diesem seinen größten Wert an, zumindest im Bereich des größten Durchmessers des Pumpenläufers, also in einem Schnitt im Bereich der Schaufelspitzen der Schaufelelemente 110.

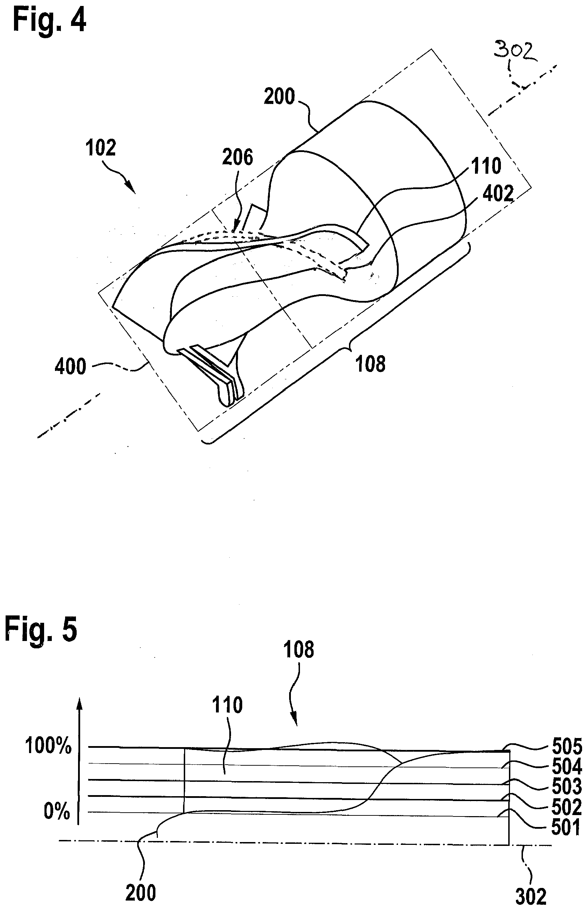

Fig. 4 zeigt eine schematische Darstellung der Axialpumpe 102 aus den Figuren 1 und 2 in perspektivischer Ansicht. Ein von dem Pumpengehäuse 104 umschlossener Bereich des Pumpenläufers 108, in dem das Pumpengehäuse 104 keine Austrittsöffnung hat, ist mit einem Rechteck 400 markiert. Gut zu erkennen ist die wellenförmige Schaufelkrümmung des Schaufelelements 110, die in einem von dem Pumpengehäuse 104 umschlossenen Bereich erstreckt ist, in dem dieses eine Austrittsöffnung 106 hat. Zum Vergleich ist neben dem Schaufelelement 110 ein herkömmliches Schaufelelement 402 ohne wellenförmige Schaufelkrümmung eingezeichnet.

Fig. 5 ist eine Ansicht des Schaufelelements der in Fig. 1 und Fig. 2 gezeigten Axialpumpe mit auf die Oberfläche der Nabe des Pumpenläufers 108 bezogenen unterschiedlichen Schaufelhöhen. Eingezeichnet sind fünf horizontale Linien, die unterschiedliche Schaufelhöhen des Schaufelelements 110 relativ zu einer Oberfläche der Nabe 200 repräsentieren. Dabei repräsentiert eine erste Linie 501 0 Prozent, eine zweite Linie 502 25 Prozent, eine dritte Linie 503 50 Prozent, eine vierte Linie 504 75 Prozent und eine fünfte Linie 505 100 Prozent einer maximalen Schaufelhöhe.

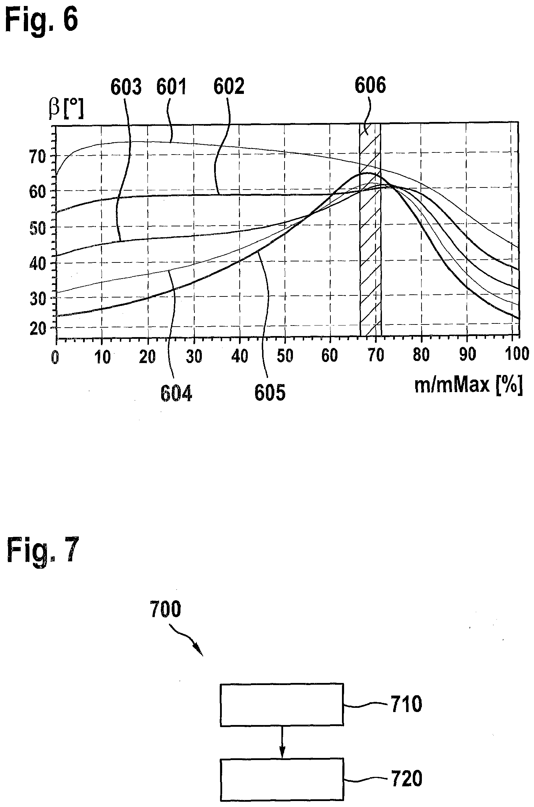

Fig. 6 zeigt ein Diagramm zur Darstellung eines Verlaufs des Schaufelwinkels β aus Fig. 3 entlang einer normierten meridionalen Koordinate m/mMax. Der Verlauf des Schaufelwinkels β ist anhand von fünf Kurven in Abhängigkeit von der Schaufelhöhe des Schaufelelements, d. h. von einem radialen Abstand dessen Skelettlinie von der Nabe, für verschiedene Schaufelschnitte dargestellt. Analog zu Fig. 5 entspricht eine erste Kurve 601 einem Schnitt des Schaufelelements entlang der ersten Linie 501 auf Höhe der Nabe bei 0 Prozent Schaufelhöhe, eine zweite Kurve 602 einem Schnitt des Schaufelelements entlang der zweiten Linie 502 bei 25 Prozent Schaufelhöhe, eine dritte Kurve 603 einem Schnitt des Schaufelelements entlang der dritten Linie 503 bei 50 Prozent Schaufelhöhe, eine vierte Kurve 604 einem Schnitt des Schaufelelements entlang der vierten Linie 504 bei 75 Prozent Schaufelhöhe und eine fünfte Kurve 605 einem Schnitt des Schaufelelements entlang der fünften Linie 505 bei 100 Prozent Schaufelhöhe, also an den Schaufelspitzen bei maximalem Laufraddurchmesser. Der Beginn des Strömungsaustritts an den Austrittsöffnungen des Pumpengehäuses ist mit einem schraffierten Bereich 606 markiert.

Aus dem Diagramm ist zum einen ersichtlich, dass der Schaufelwinkel β, und damit die Krümmung der Skelettlinie, in Strömungsrichtung je nach radialem Abstand von der Nabe unterschiedliche Verläufe aufweist. Zum anderen ist daraus ersichtlich, dass der Schaufelwinkel β in den betrachteten Schnitten jeweils in Strömungsrichtung zunächst zunimmt und ab einem Hochpunkt, der hier ein jeweiliges Maximum der Kurven bzw. der Schaufelwinkel β repräsentiert, wieder abnimmt. Die Lage des Hochpunktes entlang der meridionalen Koordinate variiert dabei je nach radialem Abstand der Skelettlinie von der Nabe.

Gemäß Fig. 6 liegt der Umkehrpunkt für die Schaufelhöhen von 25 bis 100 Prozent entweder in oder kurz vor oder kurz hinter dem schraffierten Bereich 606, dessen Abstand von der Eintrittsöffnung des Pumpengehäuses 104 in der Richtung der Drehachse 302 gesehen dem Abstand der zu der Eintrittsöffnung des Pumpengehäuses 104 weisenden Kante der Austrittsöffnung 106 des Pumpengehäuses 104 entspricht. Bezogen auf einen gleichen, auf der Drehachse 302 liegenden Koordinatenursprung haben also der die Eintrittsöffnung des Pumpengehäuses 104 zugewandte Seite der Austrittsöffnung 106 und der dem schraffierte Bereich 606 entsprechende Bereich des Pumpenläufers 108 axiale Koordinaten, die einander entsprechen, d.h. die zueinander gleich sind oder die nur wenig voneinander abweichen.

Hingegen befindet sich der Umkehrpunkt für die Schaufelhöhe von 0 Prozent deutlich vor dem schraffierten Bereich 606, hier in einem Bereich der meridionalen Koordinate zwischen 10 und 20 Prozent.

Der Pumpenläufer weist den Hochpunkt im Schaufelwinkel β im Bereich des Beginns des Strömungsaustritts über die Austrittsöffnungen oder auch in räumlicher Nähe zum Strömungsaustritt, zumindest aber im äußeren Bereich an den Schaufelspitzen auf. Es liegt in diesem Bereich also eine Änderung der Skelettlinienkrümmung von konkav nach konvex vor. Durch diese Formgebung des Schaufelelements kann der Drall in der Strömung reduziert werden, was einen effizienten Betrieb und, damit verbunden, eine schonende Fluidförderung mit reduzierter Schädigung ermöglicht.

Fig. 7 zeigt ein Ablaufdiagramm eines Verfahrens 700 zum Herstellen einer Axialpumpe gemäß einem Ausführungsbeispiel, etwa der vorangehend anhand der Figuren 1 bis 6 beschriebenen Axialpumpe. Dabei werden in einem Schritt 710 das Pumpengehäuse und der Pumpenläufer mit der Nabe und dem schraubenförmig um die Nabe gewundenen Schaufelelement gebildet. Das Schaufelelement wird dabei zumindest abschnittsweise mit einer wellenförmigen Schaufelkrümmung ausgeformt. In einem weiteren Schritt 720 erfolgt das Anordnen des Pumpenläufers in dem Pumpengehäuse. Dabei wird der Pumpenläufer um eine Drehachse drehbar gelagert, um das Fördermedium in Richtung der Drehachse zu fördern.

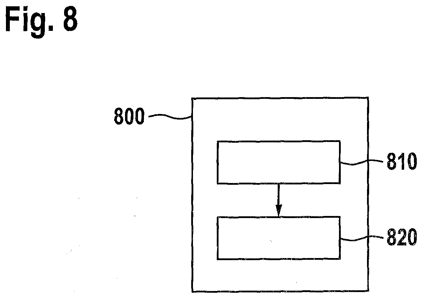

Fig. 8 zeigt eine schematische Darstellung einer Vorrichtung 800 zum Ausführen und/oder Ansteuern des Verfahrens 700 aus Fig. 7. Die Vorrichtung 800 umfasst eine erste Einheit 810 zum Bilden des Pumpengehäuses und des Pumpenläufers sowie eine zweite Einheit 820 zum Anordnen des Pumpenläufers im Pumpengehäuse.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

Zusammenfassend sind insbesondere folgende Merkmale festzuhalten: Eine Axialpumpe 102 für ein Herzunterstützungssystem umfasst ein Pumpengehäuse 104 zur Anordnung in einem Blutgefäß sowie einen in dem Pumpengehäuse 104 um eine Drehachse drehbar gelagerten oder lagerbaren Pumpenläufer 108 bestehend aus einer Nabe 200 und zumindest einem zumindest abschnittsweise schraubenförmig um die Nabe 200 gewundenen Schaufelelement 110 zum Fördern eines Fördermediums in Richtung der Drehachse 302. Zur Steigerung des Pumpenwirkungsgrades weist das Schaufelelement 110 zumindest einen Schaufelabschnitt 202 mit einer wellenförmigen Schaufelkrümmung auf.

## Patentansprüche

1. Herzunterstützungssystem für das Einführen durch ein Katheter in ein Blutgefäß,
mit einer Axialpumpe (102), die ein rohrförmiges Pumpengehäuse (104) mit einer Mantelfläche aufweist und die einen in dem Pumpengehäuse (104) erstreckten, um eine Drehachse (302) drehbaren Pumpenläufer (108) mit einer Nabe (200) und mit einem zumindest abschnittsweise schraubenförmig um die Nabe (200) gewundenen Schaufelelement (110) zum axialen Ansaugen von Blut in das Pumpengehäuse (104) in einer zu der Nabe (200) weisenden Richtung hat, wobei das Pumpengehäuse (104) eine von der Drehachse (302) durchsetzte Eintrittsöffnung hat und wenigstens eine umfangsseitige Austrittsöffnung (106) für das seitliche Ausleiten des in das Pumpengehäuse angesaugten Bluts aufweist,
**dadurch gekennzeichnet, dass**
das zumindest eine Schaufelelement (110) ein Profil mit Skelettlinien (204) aufweist, deren Krümmung bei Abwickeln in eine Ebene in der Richtung der Drehachse (302) von der Eintrittsöffnung her kommend jeweils bis zu einem Wendepunkt (310) zunimmt, in dem ein Schaufelwinkel (β) des Schaufelelements maximal ist, und deren Krümmung nach dem Wendepunkt (310) abnimmt, wobei in dem in Bezug auf die Drehachse (302) radial außenliegenden Bereich des Pumpenläufers (108), in dem für eine Schaufelhöhe SH des zumindest einen Schaufelelements (110) bezogen auf dessen maximale Schaufelhöhe SHMAX gilt: 25% ≤ SH / SHMAX ≤ 100% die Wendepunkte (310) der Skelettlinien (204) in dem Bereich einer zu der Eintrittsöffnung weisenden Kante (206) der Austrittsöffnung (106) liegen.

2. Herzunterstützungssystem nach Anspruch 1, **dadurch gekennzeichnet, dass** das Schaufelelement (110) zumindest einen Schaufelabschnitt (202) mit einer wellenförmigen Schaufelkrümmung aufweist.

3. Herzunterstützungssystem nach Anspruch 2, **dadurch gekennzeichnet, dass** der Schaufelabschnitt (202) mit der wellenförmigen Schaufelkrümmung in einem Abschnitt des Pumpenläufers (108) ausgebildet ist, der sich wenigstens teilweise in einem durch die wenigstens eine Austrittsöffnung (106) geöffneten Gehäuseabschnitt des Pumpengehäuses (104) befindet.

4. Herzunterstützungssystem nach Anspruch 2 oder 3, **dadurch gekennzeichnet, dass** die wellenförmige Schaufelkrümmung in Richtung einer radialen Ausdehnung des Schaufelabschnitts (202) variiert.

5. Herzunterstützungssystem nach einem der Ansprüche 2 bis 4, **dadurch gekennzeichnet, dass** die wellenförmige Schaufelkrümmung mit zunehmendem Abstand von der Drehachse (302) zunimmt.

6. Herzunterstützungssystem nach einem der Ansprüche 2 bis 5, **dadurch gekennzeichnet, dass** das Schaufelelement (110) in dem zumindest einen Schaufelabschnitt (202) mit der wellenförmigen Schaufelkrümmung eine in der Richtung der Drehachse (302) sich ändernde Dicke hat.

7. Herzunterstützungssystem nach einem der Ansprüche 1 bis 6, **dadurch gekennzeichnet, dass** die Nabe (200) einem distalen Ende des Herzunterstützungssystems zugewandt ist.

8. Herzunterstützungssystem nach einem der Ansprüche 1 bis 7, **dadurch gekennzeichnet, dass** die Nabe (200) einen von der Eintrittsöffnung kommend in Richtung der Drehachse (102) zunehmenden Durchmesser aufweist.

## Claims

1. Cardiac support system for insertion through a catheter into a blood vessel,
comprising an axial pump (102), which has a tubular pump housing (104) having an lateral surface, and which has a pump rotor (108) that extends in the pump housing (104), is rotatable about a rotational axis (302) and has a hub (200) and a blade element (110) wound at least partially helically around the hub (200) for axially drawing blood into the pump housing (104) in a direction pointing towards the hub (200), the pump housing (104) having an inlet opening through which the axis of rotation (302) passes and having at least one peripheral outlet opening (106) for the lateral discharge of the blood drawn into the pump housing,
**characterized in that**
the at least one blade element (110) has a profile having skeleton lines (204), the curvature of which increases when unrolled into a plane in the direction of the rotational axis (302) starting from the inlet opening up to an inflection point (310) in which a blade angle (β) of the blade element is at a maximum, and the curvature of which decreases after the inflection point (310), in the region of the pump rotor (108) lying radially on the outside in relation to the rotational axis (302), in which for a blade height SH of the at least one blade element (110), based on its maximum blade height SHMAX, the following applies: 25% ≤ SH / SHMAX ≤ 100%, the inflection points (310) of the skeleton lines (204) lying in the region of an edge (206) of the outlet opening (106) pointing to the inlet opening.

2. Cardiac support system according to claim 1, **characterized in that** the blade element (110) comprises at least one blade portion (202) having an undulating blade curvature.

3. Cardiac support system according to claim 2, **characterized in that** the blade portion (202) having the undulating blade curvature is formed in a portion of the pump rotor (108) which is at least partially in a housing portion of the pump housing (104) that is opened by the at least one outlet opening (106).

4. Cardiac support system according to either claim 2 or claim 3, **characterized in that** the undulating blade curvature varies in the direction of a radial extension of the blade portion (202).

5. Cardiac support system according to any of claims 2 to 4, **characterized in that** the undulating blade curvature increases with increasing distance from the rotational axis (302).

6. Cardiac support system according to any of claims 2 to 5, **characterized in that**, in the at least one blade portion (202) having the undulating blade curvature, the blade element (110) has a thickness which changes in the direction of the rotational axis (302).

7. Cardiac support system according to any of claims 1 to 6, **characterized in that** the hub (200) faces a distal end of the cardiac support system.

8. Cardiac support system according to any of claims 1 to 7, **characterized in that** the hub (200) has a diameter which increases starting from the inlet opening in the direction of the rotational axis (302).

## Revendications

1. Système d'assistance ventriculaire destiné à être inséré dans un vaisseau sanguin à travers un cathéter,
comportant une pompe axiale (102) qui présente un carter de pompe tubulaire (104) comportant une surface externe et qui possède un rotor de pompe (108) s'étendant dans le carter de pompe (104), rotatif autour d'un axe de rotation (302) et comportant un moyeu (200) et comportant un élément d'aube (110) enroulé de manière hélicoïdale, au moins dans certaines régions, autour du moyeu (200) afin d'aspirer axialement du sang dans le carter de pompe (104) dans un sens pointant vers le moyeu (200), le carter de pompe (104) possédant une ouverture d'entrée traversée par l'axe de rotation (302) et présentant au moins une ouverture de sortie (106) côté circonférence pour l'évacuation latérale du sang aspiré dans le carter de pompe,
**caractérisé en ce que**
l'au moins un élément d'aube (110) présente un profil comportant des lignes de squelette (204) dont la courbure augmente lorsqu'il est déroulé dans un plan dans le sens de l'axe de rotation (302) en provenance de l'ouverture d'entrée respectivement jusqu'à un point d'inflexion (310) au niveau duquel un angle d'aube (β) de l'élément d'aube est maximal, et sa courbure diminue après le point d'inflexion (310), les points d'inflexion (310) des lignes de squelette (204) se trouvant dans la zone d'un bord (206) de l'ouverture de sortie (106) pointant vers l'ouverture d'entrée dans la zone du rotor de pompe (108) située radialement à l'extérieur par rapport à l'axe de rotation (302), dans laquelle zone s'applique, pour une hauteur d'aube SH de l'au moins un élément d'aube (110), par rapport à sa hauteur d'aube maximale SHMAX : 25 % ≤ SH / SHMAX ≤ 100%.

2. Système d'assistance ventriculaire selon la revendication 1, **caractérisé en ce que** l'élément d'aube (110) présente au moins une section d'aube (202) comportant une courbure d'aube ondulée.

3. Système d'assistance ventriculaire selon la revendication 2, **caractérisé en ce que** la section d'aube (202) comportant la courbure d'aube ondulée est formée dans une section du rotor de pompe (108) qui se trouve au moins partiellement dans une section de carter du carter de pompe (104) ouverte par l'au moins une ouverture de sortie (106).

4. Système d'assistance ventriculaire selon la revendication 2 ou 3, **caractérisé en ce que** la courbure d'aube ondulée varie dans le sens d'une expansion radiale de la section d'aube (202).

5. Système d'assistance ventriculaire selon l'une des revendications 2 à 4, **caractérisé en ce que** la courbure d'aube ondulée augmente lorsque la distance depuis l'axe de rotation (302) augmente.

6. Système d'assistance ventriculaire selon l'une des revendications 2 à 5, **caractérisé en ce que** l'élément d'aube (110) possède une épaisseur qui varie dans le sens de l'axe de rotation (302) dans l'au moins une section d'aube (202) comportant la courbure d'aube ondulée.

7. Système d'assistance ventriculaire selon l'une des revendications 1 à 6, **caractérisé en ce que** le moyeu (200) fait face à une extrémité distale du système d'assistance ventriculaire.

8. Système d'assistance ventriculaire selon l'une des revendications 1 à 7, **caractérisé en ce que** le moyeu (200) présente un diamètre qui augmente, en provenance de l'ouverture d'entrée, dans le sens de l'axe de rotation (102).
